# EUROPEAN PATENT SPECIFICATION

(11) **EP 2 959 892 B1**
(45) Date of publication and mention of the grant of the patent: **09.09.2020**
(21) Application number: 14754975.2
(22) Date of filing: 21.02.2014
(51) Int. Cl.: A61K 9/28, A61K 9/32, A61K 9/36, A61K 31/606, A61P 1/04

(54) **ENTERIC COATED TABLET**
MAGENSAFTRESISTENTE TABLETTE
COMPRIMÉ ENROBÉ GASTRO-RÉSISTANT

(30) Priority: 22.02.2013 JP 2013032759
(43) Date of publication of application: 30.12.2015
(73) Proprietor: Zeria Pharmaceutical Co., Ltd., Tokyo 103-8351 (JP)
(72) Inventor: RYU, Akio, Tokyo 103-8351 (JP); OSADA, Miyako, Tokyo 103-8351 (JP)
(74) Representative: Blodig, Wolfgang
(86) International application number: PCT/JP2014/054104
(87) International publication number: WO 2014/129568

(56) References cited:
- WO-A1-03/045356
- WO-A1-2012/028698
- WO-A1-2012/028698
- WO-A1-2013/164316
- WO-A2-2009/047802
- WO-A2-2011/012990
- JP-A- S58 109 413
- JP-A- S58 109 413
- JP-A- 2003 501 458
- JP-A- 2011 500 553
- US-A1- 2009 162 434
- US-A1- 2009 162 434
- US-A1- 2010 129 446
- US-B1- 6 773 720

## Description

### Technical Field

The present invention relates to an enteric coated tablet.

### Background Art

Some medicinal ingredients of pharmaceutical formulations are not stable under acidic conditions. When a conventional drug product containing such an ingredient is orally administered, the ingredient cannot fully exhibit a pharmaceutical effect of interest, due to degradation by gastric acid or the like.

Meanwhile, in the case of a therapeutic agent for an inflammatory bowel disease such as ulcerative colitis or Crohn's disease, a medicinal ingredient is preferably released in the intestinal tract; i.e., in an affected organ. One form of the pharmaceutical formulation containing such a medicinal ingredient is an enteric drug product, such as an enteric coated tablet.

Specifically, 5-aminosalicylic acid (mesalazine) and salazosulfapyridine, which are therapeutic ingredients for inflammatory bowel diseases, are commercially marketed in various dosage forms such as an enteric drug product, an enema agent, and a suppository, and are widely used for the relevant therapy. Among such formulations, an enteric coated tablet is most generally used, by virtue of excellent oral availability, portability, ease of quality control, etc. Examples of such commercially available enteric coated tablet products include an enteric coated tablet which is dissolved in the second fluid for dissolution test (pH: 6.8) of the Japanese Pharmacopoeia and an enteric coated tablet which is dissolved at pH 7 or higher. Among them, an enteric coated tablet which is designed to be dissolved at pH 7 or higher is particularly useful for colonic diseases, since the tablet releases a medicinal ingredient when it has reached the lower gastrointestinal tract (from terminal ileum to colon); i.e., an affected organ.

Polaprezinc, which exhibits gastric mucosa protecting effect and cell protecting effect, has already been used as therapeutic drugs for gastric ulcer and duodenal ulcer. Recently, research has been conducted to use the drug as an enema for ulcerative colitis and disorder of the rectal mucosa (Non-patent Documents 1 to 4). In a enema therapy of polaprezinc, an endoscopically significant improvement was observed only in a region where the enema had reached. Thus, it is obviously difficult for conventional oral administration drug products to reach a lower part of the colon. Accordingly, for effective treatment of ulcerative colitis and disorder of the rectal mucosa through use of polaprezinc, an enteric coated tablet of a large size which reaches the colon without fail and sufficiently acts on an affected area has been demanded from the viewpoint of drug compliance.

Another technique in relation to an enteric coating has also been reported. The technique is pH-dependent double layer enteric coating (Patent Documents 1 and 2).

### Citation List

### Patent Documents

[Patent Document 1] JP-A-2001-502333
[Patent Document 2] JP-A-2005-510539 (family member: WO 03/045356)

### Non-patent Documents

[Non-patent Document 1] http://informahealthcare.com/doi/ abs/10.3109/00365521.2013.863963
[Non-patent Document 2] http://ir.jikei.ac.jp/bitstream/ 10328/7851/1/KKN2011-89.pdf
[Non-patent Document 3] The Journal of JASTRO 21(3/4): 149-154 2009
[Non-patent Document 4] http://kaken.nii.ac.jp/pdf/2011/ seika/C-19/34519/21591622seika.pdf

### Summary of the Invention

### Problems to be Solved by the Invention

In the case of a 5-aminosalicylic acid, three enteric coated tablets each containing 400 mg of 5-aminosalicylic acid must be administered as a unit dose. Therefore, a tablet containing a unit dose of 5-aminosalicylic acid is desired. However, when a huge enteric coated tablet containing a medicinal ingredient in a large amount and having enough impact resistance is manufactured, the thickness of the enteric coating layer must be increased. In this case, drug release is problematically delayed. In contrast, when the thickness of the coating layer or the amount of diluent is reduced, acid resistance is poor, and impact resistance is impaired, which is problematic.

Under such circumstances, an object of the present invention is to provide an enteric coated tablet containing a large amount of medicinal ingredient and having sufficient impact resistance, without forming a thick enteric coating. Means for Solving the Problems

The present inventors previously conducted extensive studies on the relationship between the composition of the coating, and the enteric property and impact resistance of an enteric coated tablet containing a large amount of active ingredient. As a result, when referring to Patent Documents 1 and 2, a large number of tablets were broken during a coating process of double enteric layers; and a coating process of triple layers for improving impact resistance made the steps cumbersome and prolonged the processing time. Thus, these techniques were difficult to apply for the industrial production of huge tablets, from technical and economical viewpoints. Furthermore, each enteric coating layer must be appropriately controlled in coating process, making control of enteric properties difficult. Under such circumstances, the present inventors have conducted further studies, and have found that, by providing a double layer coated tablet including a water-soluble polymer coating layer and an enteric coating layer, with the water-soluble polymer coating layer serving as an inner layer, and regulating the total amount and each amount of two coating layers within specific ranges, both consistent drug release characteristics in the lower gastrointestinal tract and high impact resistance can be attained. The present invention has been accomplished on the basis of this finding.

Accordingly, the present invention provides the following [1] to [10]:
[1] an enteric coated tablet, comprising (A) a core tablet containing a medicinal ingredient and having a weight of 1,000 mg or more; (B) a coating layer containing a water-soluble polymer applied onto the surface of the core tablet, the water-soluble polymer coating (B) having no enteric property; and (C) an enteric coating layer dissolving at pH 7 or higher which is applied onto the surface of the coating layer containing a water-soluble polymer, wherein the sum of the polymer amount of the coating layer (B) and the polymer amount of the coating layer (C) is 10 to 18 mg/cm², the polymer amount of the coating layer (B) is 6 to 12 mg/cm², and the polymer amount of the coating layer (C) is 3 to 6 mg/cm²;
[2] the enteric coated tablet according to [1] above, wherein the core tablet (A) has a weight of 1,000 to 1,500 mg;
[3] the enteric coated tablet according to [1] or [2] above, which has a medicinal ingredient content of 700 to 1,300 mg;
[4] the enteric coated tablet according to any one of [1] to [3] above, wherein the water-soluble polymer is one or more members selected from the group consisting of water-soluble cellulose ethers;
[5] the enteric coated tablet according to any one of [1] to [4] above, wherein the enteric coating layer dissolving at pH 7 or higher is a coating layer formed of one or more polymers selected from the group consisting of methacrylic acid copolymers.
[6] the enteric coated tablet according to any one of [1] to [5] above, which has a total weight of 1,200 to 1,600 mg;
[7] the enteric coated tablet according to any one of [1] to [6] above, wherein the medicinal ingredient is a therapeutic agent for a colonic disease.
[8] the enteric coated tablet according to any one of [1] to [6] above, wherein the medicinal ingredient is a therapeutic agent for an inflammatory bowel disease.
[9] the enteric coated tablet according to any one of [1] to [6] above, wherein the medicinal ingredient is 5-aminosalicylic acid; and
[10] the enteric coated tablet according to any one of [1] to [9] above, which has a ratio by polymer mass (B/C) of the coating layer (B) to the coating layer (C) of 1.0 to 2.5.

### Effects of the Invention

Even though the enteric coated tablet of the present invention contains a medicinal ingredient in a large amount per tablet, excellent release properties in the lower gastrointestinal tract, and high impact resistance are attained. Thus, pathological condition which has conventionally required a plurality of tablets as a single dose for the therapy can be treated with one tablet of the drug product as a single dose.

### Brief Description of the Drawings

[Fig. 1] A graph showing the results of a drop test (height: 60 cm) of coated tablets.
[Fig. 2] A graph showing the relationship between outer coating polymer amount and lag time in a dissolution test using intestinal juice (pH: 7.2).

### Modes for Carrying Out the Invention

The enteric coated tablet of the present invention comprises (A) a core tablet containing a medicinal ingredient and having a weight of 1,000 mg or more; (B) a coating layer containing a water-soluble polymer applied onto the surface of the core tablet; and (C) an enteric coating layer dissolving at pH 7 or higher which is applied onto the surface of the coating layer containing a water-soluble polymer, wherein the sum of the polymer amount of the coating layer (B) and the polymer amount of the coating layer (C) is 10 to 18 mg/cm², the polymer amount of the coating layer (B) is 6 to 12 mg/cm², and the polymer amount of the coating layer (C) is 3 to 6 mg/cm².

The core tablet (A) employed in the enteric coated tablet of the present invention is a core tablet (i.e., an uncoated tablet) containing a medicinal ingredient and having a weight of 1,000 mg or more. The medicinal ingredient is preferably a drug substance which is released in the intestinal tract, and examples of preferred drug substances include a non-steroidal anti-inflammatory agent, an ulcer therapeutic agent, an anti-bacterial agent, a peptide, a protein, and a steroid. Among them, drug substances which are released in the lower gastrointestinal tract (from terminal ileum to colon) are particularly preferred; specifically, therapeutic drug substances for inflammatory bowel disease, colon cancer, etc. are preferred. Examples of preferred drug substances which act on the colon include 5-aminosalicylic acid (mesalazine), salazosulfapyridine, prednisolone, betamethasone, and polaprezinc. Of these, 5-aminosalicylic acid is more preferred. 5-Aminosalicylic acid is used as a therapeutic agent for an inflammatory bowel disease such as ulcerative colitis or Crohn's disease.

The weight of the core tablet (A) is preferably 1,000 mg or more. The weight is more preferably 1,000 to 1,500 mg, even more preferably 1,200 to 1,500 mg, from the viewpoints of ease of taking and portability. The content of the medicinal ingredient in the core tablet (A) is preferably 700 mg or more, more preferably 900 to 1,300 mg, even more preferably 1,000 to 1,300 mg, from the viewpoints of ease of taking, good impact resistance, portability, and usage.

In addition to the medicinal ingredient(s), the core tablet (A) may further contain additives which are generally incorporated into tablets, such as diluents, disintegrants, lubricants, binders, glidants, flavoring agents, odor improving agents, and plasticizers. The total amount of the additives contained in the core tablet (A) is preferably 100 to 600 mg, more preferably 150 to 400 mg, even more preferably 200 to 400 mg. Examples of the diluent include a saccharide (e.g., lactose, sucrose, glucose, or mannitol), starch, partially pregelatinized starch, crystalline cellulose, calcium carbonate, calcium sulfate, and sodium hydrogencarbonate. Of these, lactose, mannitol, starch, and crystalline cellulose are preferred, with crystalline cellulose being more preferred. Examples of the disintegrant include starch, agar, gelatin powder, crystalline cellulose, carmellose sodium, carmellose calcium, croscarmellose sodium, crospovidone, carcium carbonate, sodium hydrogencarbonate, sodium starch glycolate, hydroxypropylmethylcellulose, and low-substituted hydroxypropylcellulose. Of these, croscarmellose sodium, crospovidone, sodium starch glycolate, and low-substituted hydroxypropylcellulose are preferred, with sodium starch glycolate being more preferred. Examples of the lubricant include magnesium stearate, calcium stearate, talc, hydrogenated vegetable oil, macrogol, and sodium stearyl fumarate. Of these, calcium stearate and talc are preferred, with magnesium stearate being more preferred. Examples of the binder include starch, gum arabic, gelatin, sodium alginate, methylcellulose, ethylcellulose, povidone, polyvinyl alcohol, hydroxypropylcellulose, and carboxymethylcellulose. Of these, povidone, hydroxypropylcellulose, and hypromellose are preferred, with povidone and hypromellose being more preferred. If required, a glidant may be added. Examples of the glidant include silicon dioxides such as light anhydrous silicic acid.

The core tablet (A) may be manufactured through a conventional method known in the art, and any of direct powder compaction, dry granule compaction, semi-dry granule compaction, and wet granule compaction may be employed. Among these techniques, a combination of wet granulation and subsequent dry compaction is preferred.

The core tablet (A) preferably has a hardness (in the longitudinal direction) of 160 N or higher, more preferably 170 N or higher, even more preferably 180 N or higher, from the viewpoint of impact resistance. More specifically, the hardness of the core tablet (A) preferably falls within a range of 160 to 300 N, more preferably within a range of 170 to 300 N, from the viewpoint of impact resistance. The hardness may be measured by a hardness tester (for example, PTB302 produced by PHARMA TEST).

The enteric coated tablet of the present invention comprises a water-soluble polymer coating layer (B) on the surface of the aforementioned core tablet (A). The coating layer (B) is a water-soluble polymer coating layer having no enteric property, and is a conventional coating layer containing a water-soluble polymer as a main ingredient. Examples of the water-soluble polymer employed in the coating layer (B) include water-soluble cellulose ethers such as hydroxypropylcellulose, hydroxypropylmethylcellulose (hypromellose), and hydroxyethylcellulose; polyethylene glycol; gelatin; alginate salts; dextrin; and water-soluble polyvinyl alcohol derivatives such as polyvinyl alcohol-acrylic acid-methyl methacrylate copolymer and polyvinyl alcohol-polyethylene glycol graft copolymer. Of these, water-soluble cellulose ethers are preferred, with one or more species selected from hydroxypropylcellulose and hydroxypropylmethylcellulose being more preferred.

The coating layer (B) may be formed by spraying a solution containing the aforementioned water-soluble polymer onto the core tablet (A), and then drying the tablet. The solution containing the water-soluble polymer may further contain a plasticizer such as triethyl citrate, polysolvate, or polyethylene glycol. Examples of the solvent of the solution include water, ethanol, and a water-ethanol mixture.

The enteric coated tablet of the present invention comprises, on the surface of the coating layer (B), an enteric coating layer (C) dissolving at pH 7 or higher. When dissolving at pH 7 or higher, the coating layer (C) allows a medicinal ingredient in the enteric coated tablet of the present invention to be released at the lower gastrointestinal tract. The polymer employed in the coating layer (C) to form the enteric coating dissolving at pH 7 or higher is preferably methacrylic acid copolymers, more preferably methacrylic acid-methyl methacrylate copolymer, even more preferably one or more species selected from methacrylic acid copolymer L, methacrylic acid copolymer S, and methacrylic acid copolymer LD. Even more preferably, one or more species selected from methacrylic acid copolymer L and methacrylic acid copolymer S are used.

The coating layer (C) may be formed by spraying a solution containing the aforementioned polymer dissolving at pH 7 or higher onto the tablet coated with the coating layer (B), and then drying the resultant tablet. The solution containing a polymer dissolving at pH 7 or higher may further contain a plasticizer such as triethyl citrate, a lubricant such as talc, a pigment, or the like. Examples of the solvent of the solution include water, ethanol, isopropanol, acetone, and a mixture thereof.

In the enteric coated tablet of the present invention, the sum of the polymer amount of the coating layer (B) and the polymer amount of the coating layer (C) is 10 to 18 mg/cm², the polymer amount of the coating (layer B) is 6 to 12 mg/cm², and the polymer amount of the coating layer (C) is 3 to 6 mg/cm². These conditions are important from the viewpoint of attaining favorable enteric property and excellent impact resistance.

When the sum of the polymer amount of the coating layer (B) and the polymer amount of the coating layer (C) is less than 10 mg/cm², impact resistance is poor, whereas when the sum of the amounts is in excess of 18 mg/cm², the time required for tablet manufacturing and dissolution lag time increase, thereby failing to attain appropriate drug release. Thus, the sum of the amounts is preferably 10 to 16 mg/cm², more preferably 10 to 15 mg/cm².

When the polymer amount of the coating layer (B) is less than 6 mg/cm², impact resistance is poor, whereas when the amount is in excess of 12 mg/cm², the relative polymer amount of the coating layer (C) decreases, thereby making control of release characteristics difficult. Thus, the polymer amount of the coating layer (B) is preferably 6 to 11 mg/cm², more preferably 6 to 10 mg/cm².

When the polymer amount of the coating layer (C) is less than 3 mg/cm², difficulty is encountered in controlling release characteristics, whereas when the amount is in excess of 6 mg/cm², the relative polymer amount of the coating layer (B) decreases, thereby failing to attain sufficient impact resistance. Thus, the polymer amount of the coating layer (C) is preferably 4 to 6 mg/cm².

The ratio by polymer mass (B/C) of the coating layer (B) to the coating layer (C) is preferably 1.0 to 2.5, more preferably 1.5 to 2.5, from the viewpoints of industrial production, impact resistance, and control of release properties.

The total weight of the enteric coated tablet of the present invention is preferably 1,060 to 1,650 mg, more preferably 1300 to 1600 mg, even more preferably 1350 to 1550 mg, from the viewpoint of ease of taking.

The enteric coated tablet of the present invention preferably has a hardness (in the longitudinal direction) of 250 N or more, more preferably 260 N or more, from the viewpoint of impact resistance. More specifically, the hardness of the enteric coated tablet preferably falls within a range of 250 to 500 N, more preferably within a range of 260 to 450 N, from the viewpoint of impact resistance. The hardness may be measured by a hardness tester (for example, PTB502 produced by PHARMA TEST).

The enteric coated tablet of the present invention may be in the shape of regular disk or oval and may be a roundish tablet having one or more radii of curvature. The aspect ratio of the enteric coated tablet of the invention is preferably 1 : 1 to 22 : 9, more preferably 20 to 21 : 9 to 10, from the viewpoint of impact resistance. The thickness of the tablet is preferably 6 to 8 mm, more preferably 6.5 to 8 mm, more preferably 6.5 to 7.8 mm, even more preferably 7.2 to 7.6 mm.

### Examples

The present invention will next be described in detail by way of examples, which should not be construed as limiting the invention thereto.

### (Referential Example 1)

5-Aminosalicylic acid (active ingredient) (85.7%), crystalline cellulose (about 8.5 to about 9.5%), sodium starch glycolate (3%), and hypromellose (1.5 to 2.5%) were mixed and wet-granulated, and the granulated product was dried and mixed with magnesium stearate (about 0.3%). The mixture was compressed by a rotary tablet press, to thereby manufacture tablets each weighing about 1,400 mg and containing 1,200 mg of active ingredient. The longer diameter and shorter diameter of each tablet are 20.5 mm and 9.5 mm, respectively. The mean value of the core tablet hardness was about 240 N (measured by a hardness tester PTB302 or PTB502, product of PHARMA TEST).

### (Comparative Example 1)

A coating solution having a composition shown in the following table was sprayed onto core tablets manufactured in Referential Example 1, to thereby form, on each tablet, an outer coating layer of methacrylic acid copolymer S.

**[Table 1]**

| Ingredients | (mass%) |
|---|---|
| Methacrylic acid copolymer S | 7.0 |
| Triethyl citrate | 1.4 |
| Talc | 1.0 |
| Pigment | 0.8 |
| Isopropanol | 63.9 |
| Acetone | 21.9 |
| Purified water | 4.0 |

Coating was performed by a pan coater (drum capacity: about 10 L). The results are shown in Table 2.

**[Table 2]**

| Test results (no inner coating layer) | | | | |
|---|---|---|---|---|
| (Number of passed products/Number of tested products) | | | | |
| Test items | | Acid resistance (pH 1.2, 2 hr) | Drop test (30 cm×10) | Percent tablet breakage (%) |
| Amount of methacrylic acid copolymer S of outer layer (mg/cm²) | 4 | 0/3 | 1/10 | 26.6 |
| | 6 | 2/3 | 1/10 | 9.5 |
| | 8 | 2/3 | 2/10 | 5.1 |

| | | | | |
|---|---|---|---|---|
| Tablet breakage: breakage resulting in core tablet exposure, coating film crack | | | | |

The coating layer formed of a methacrylic acid copolymer is rigid but is poor in elasticity and plasticity. Thus, in the case of very huge tablets, collision between tablets and collision of tablets with the coating pan occurred with high impact during rolling of the coating pan. Even in the case of small-scale manufacturing, a number of tablets were problematically broken (resulting in core tablet exposure, coating film cracking, etc.).

Among the manufactured coated tablets, those having good appearance were subjected to the disintegration test defined by the Japanese Pharmacopoeia. Specifically, the tablets were tested for two hours in the first fluid (pH: 1.2). After completion of the test, the appearance of each tablet was observed. Among three 4 mg/cm²-coated tablets, all tablets experienced coating damage, and among three 8 mg/cm²-coated tablets, one tablet experienced coating damage. Thus, the two series of tablets were found to have acid resistance lower than the level required for enteric drug products. This test result has revealed problems occurring when a huge core tablet is directly coated with an enteric coating layer formed of a methacrylic acid copolymer.
Specifically, a uniform coating layer fails to be formed, to thereby increase variation in quality of tablets, and tablets which have no problematic appearance but fail to meet quality standards may tend to be manufactured. In addition, difficulty is encountered in directly applying the technique disclosed in Patent Document 2 to manufacturing of huge tablets.

In a drop test, 10 tablets of each type were caused to fall, repeatedly 10 times, into a stainless steel container from a height of 30 cm from the bottom of the container. Through observation of appearance of the tablets after completion of the drop test, the percentage of good products was as low as about 10 to about 20%. Accordingly, the coating method in which a core tablet is directly coated with the methacrylic acid copolymer layer was thought to be an impractical method, in consideration of mechanical stress encountered in a coating step and a package step on an actual manufacturing scale, conveyance, and routine handling.

### (Referential Example 2)

A coating solution having a composition shown in the following table was sprayed onto core tablets manufactured in Referential Example 1, to thereby form, on each tablet, an inner coating layer of hypromellose.

**[Table 3]**

| Ingredients | (mass%) | |
|---|---|---|
| Hypromellose | 6.0 | 9.0 |
| Triethyl citrate | 1.2 or 1.8 | |
| Purified water or purified water-anhydrous ethanol mixture | 92.8 | 89.2 |

### (Example 1)

To each tablet having a hypromellose inner coating layer thereon manufactured in Referential Example 2, a coating solution having a composition of Table 1 was sprayed, to thereby form an outer coating layer of methacrylic acid copolymer S.

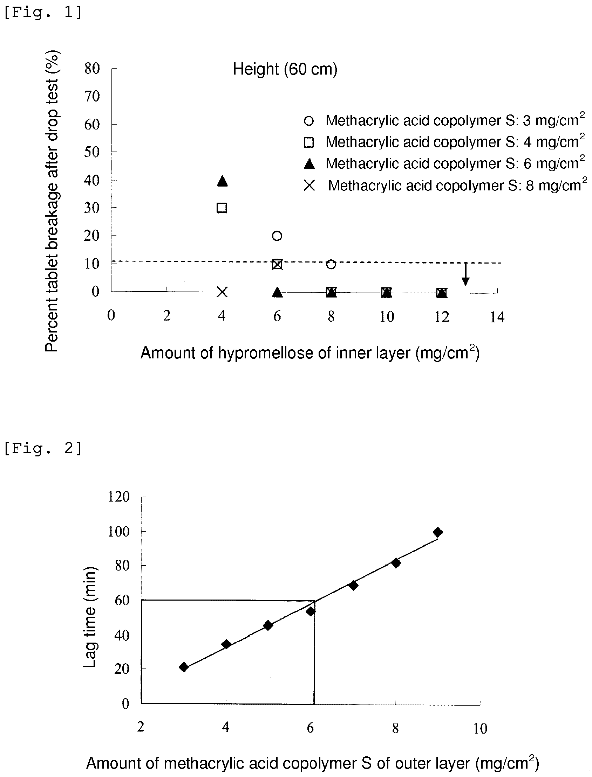

Coating was performed by a pan coater (drum capacity: about 10 L). In a drop test, 10 tablets of each type were caused to fall, repeatedly 10 times, into a stainless steel container from a specific height from the bottom of the container, and the appearance of each tablet was observed after the drop test. When the amount of inner coating (hypromellose) was 4 to 8 mg/cm² with respect to the surface area of the core tablet, impact resistance increased with the coating amount. In the case where the amount of methacrylic acid copolymer S forming the outer layer was 4 mg, when the amount of hypromellose forming the inner layer was 6 mg/cm² or more, or in the case where the amount of methacrylic acid copolymer S forming the outer layer was 3 mg, when the amount of hypromellose forming the inner layer was 8 mg/cm² or more, sufficient impact resistance was attained (Table 4 and Fig. 1). The mean value of the tablets hardness measured by a hardness tester PTB502 (product of PHARMA TEST) were about 370 N and about 400 N, respectively.

**[Table 4]**

| Results of drop test (10 tablets, 10 times, Number of passed products/Number of tested products) | | | | | | | | | | | |
|---|---|---|---|---|---|---|---|---|---|---|---|
| | | Amount of hypromellose of inner layer (mg/cm²) | | | | | | | | | |
| | | 4 | | 6 | | 8 | | 10 | | 12 | |
| Drop height | | 30 cm | 60 cm | 30 cm | 60 cm | 30 cm | 60 cm | 30 cm | 60 cm | 30 cm | 60 cm |
| Amount of methacrylic acid copolymer S of outer layer (mg/cm²) | 3 | - | - | 9/10 | 8/10 | 10/10 | 9/10 | - | - | - | - |
| | 4 | 9/10 | 7/10 | 10/10 | 9/10 | 10/10 | 10/10 | 10/10 | 10/10 | 10/10 | 10/10 |
| | 6 | 10/10 | 6/10 | 10/10 | 10/10 | 10/10 | 10/10 | 9/10 | 10/10 | 10/10 | 10/10 |
| | 8 | 10/10 | 10/10 | 10/10 | 9/10 | 10/10 | 10/10 | 10/10 | 10/10 | 10/10 | 10/10 |
| | 12 | - | - | - | - | 10/10 | 9/10 | - | - | - | - |
| | 15 | - | - | - | - | 10/10 | 9/10 | - | - | - | - |
| | 18 | - | - | - | - | 10/10 | 10/10 | - | - | - | - |

The thus-manufactured coated tablets were subjected to the disintegration test defined by the Japanese Pharmacopoeia employing the first fluid (pH: 1.2) for two hours, and the appearance of each tablet was observed after the disintegration test. In the case where the amount of hypromellose forming the inner layer was 4 mg/cm², when the amount of methacrylic acid copolymer S was 4 mg/cm² or more, or in the case where the amount of hypromellose forming the inner layer was 6 mg/cm², when the amount of methacrylic acid copolymer S was 3 mg/cm² or more, a sufficient level of acid resistance required for enteric drug products was ensured (Table 5).

**[Table 5]**

| Results of acid resistance test (pH 1.2, 2 hr) | | | | |
|---|---|---|---|---|
| (Number of passed products/Number of tested products) | | | | |
| | | Amount of hypromellose of inner layer (mg/cm²) | | |
| | | 4 | 6 | 8 |
| Amount of methacrylic acid copolymer S of outer layer (mg/cm²) | 3 | - | 3/3 | 3/3 |
| | 4 | 3/3 | 3/3 | 3/3 |
| | 6 | 3/3 | 3/3 | 3/3 |
| | 8 | 3/3 | - | - |

In the case of a pH-dependent controlled release drug product, which can control the medicinal ingredient release target area of the gastrointestinal tract by pH variation therein, the drug product is required to release the medicinal ingredient via rapid dissolution of the coating, when the drug product is exposed to the pH environment of the target gastrointestinal tract area. Fig. 2 is a graph showing the lag time (the period of time required for medicial ingredient release to begin) of a coated tablet in the dissolution test employing diluted Mcllvine buffer (pH: 7.2). As is clear from the graph, the lag time of the coated tablet is prolonged considerably depending on the amount of methacrylic acid copolymer S. In the case of the above tablet, when the amount was in excess of 6 mg/cm², the lag time was 60 minutes or longer. Therefore, the amount of methacrylic acid copolymer S of the outer coating layer is thought to be preferably 3 to 6 mg/cm².

In consideration of actual production, the coating process was conducted by a pan coater having a drum capacity of about 550 L. The spraying times required for forming the layers are shown in Table 6. The spraying time is preferably 6 hours or less for completion of the tablet manufacturing process within a day, the process including preparation of a coating solution and drying after spraying. Thus, the upper limit of the inner coating amount (as reduced to the amount of hypromellose) was adjusted to 12 mg/cm².

**[Table 6]**

| Charge amount (140 kg) | | Spraying time | |
|---|---|---|---|
| | | Inner layer | Outer layer |
| Coating amount^{*1} (mg/cm²) | 3 | | 70 min |
| | 4 | 125 min | 95 min |
| | 6 | 180 min | 145 min |
| | 8 | 240 min | 195 min |
| | 10 | 300 min | 245 min |
| | 12 | 360 min | 295 min |

| | | | |
|---|---|---|---|
| *1: as hypromellose or methacrylic acid copolymer S | | | |

From the aforementioned results including impact resistance, acid resistance, enteric performance, and spraying time, the sum of the polymer amount of the coating layer (B) (inner coating layer (water-soluble polymer)) and the polymer amount of the coating layer (C) (outer coating layer (coating layer dissolving at pH 7 or higher)) is preferably 10 to 18 mg/cm², the polymer amount of the coating layer (B) is preferably 6 to 12 mg/cm², and the polymer amount of the coating layer (C) is preferably 3 to 6 mg/cm².

### (Example 2)

Core tablets (about 140 kg) manufactured in Referential Example 1 were added to a pan coater (drum capacity: about 550 L). To each tablet, a coating solution having a composition of Table 7 was sprayed, to thereby form an inner coating layer of hypromellose so that the amount of hypromellose was adjusted to 8 mg/cm² with respect to the surface area of the core tablet.

**[Table 7]**

| Ingredients | (%) |
|---|---|
| Hypromellose | 9.0 |
| Triethyl citrate | 1.8 |
| Anhydrous ethanol | 53.5 |
| Purified water | 35.7 |

Subsequently, to each of the aforementioned tablets having a hypromellose inner coating layer thereon, a coating solution having a composition of Table 1 was sprayed, to thereby form an outer coating layer of methacrylic acid copolymer S so that the amount of methacrylic acid copolymer S was adjusted to 4 mg/cm² with respect to the surface area of the core tablet. The mean value of the tablets hardness measured by a hardness tester PTB502 (product of PHARMA TEST) was about 450 N.

As is clear from Table 8 showing the test results, the thus-manufactured coated tablets were found to have a sufficient level of acid resistance required for enteric drug products and improved impact resistance. In manufacturing of the coated tablets on a production scale, the percentage of defective (broken) tablets was less than 0.1%, indicating that the tablets facilitate the efficient manufacture of the drug products to a high degree.

**[Table 8]**

| Test results of coated tablets | | | |
|---|---|---|---|
| Acid resistance test (pH: 1.2, 2 hr) | Drop test | | Percent tablet breakage (%) |
| | (30 cm × 10 | (60 cm × 10 | |
| 3/3 | 10/10 | 10/10 | 0.095 |

| | | | |
|---|---|---|---|
| Number of passed products/Number of tested products Tablet breakage: breakage resulting in core tablet exposure, coating film crack | | | |

### (Referential Example 3)

5-Aminosalicylic acid (active ingredient) (85.7%) and hypromellose (about 2%) were mixed and wet-granulated. The granulated product was dried and then mixed with crystalline cellulose (about 9.2%), sodium starch glycolate (about 3%), and magnesium stearate (about 0.5%). The mixture was compressed by a rotary tablet press, to thereby manufacture tablets each weighing about 1,400 mg and containing 1,200 mg of active ingredient. The longer diameter and shorter diameter of each tablet are 21 mm and 10 mm, respectively. The mean value of the core tablets hardness measured by a hardness tester PTB302 (product of PHARMA TEST) was about 170 N.

### (Example 3)

A coating solution having a composition of Table 3 was sprayed onto core tablets manufactured in Referential Example 1, to thereby form, on each tablet, an inner coating layer so that the amount of hypromellose was adjusted to 6 mg/cm² with respect to the surface area of the core tablet.

Subsequently, to the thus-manufactured tablets having a hypromellose inner coating layer thereon, a coating solution having a composition of Table 9 was sprayed, to thereby form, on each tablet, an outer coating layer so that the total amount of methacrylic acid copolymers S and L was adjusted to 6 mg/cm² with respect to the surface area of the core tablet. The mean value of the resultant tablets hardness measured by a hardness tester PTB502 (product of PHARMA TEST) was about 280 N to about 320 N.

As is clear from Table 10 showing the test results, the thus-manufactured coated tablets were found to have a sufficient level of acid resistance required for enteric drug products and to exhibit good impact resistance.

**[Table 9]**

| Ingredients | (mass%) | | | | |
|---|---|---|---|---|---|
| Methacrylic acid copolymer S | 5.6 | or | 4.9 | or | 4.2 |
| Methacrylic acid copolymer L | 1.4 | | 2.1 | | 2.8 |
| Triethyl citrate | 1.4 | | 1.4 | | 1.4 |
| Talc | 1.0 | | 1.0 | | 1.0 |
| Pigment | 0.8 | | 0.8 | | 0.8 |
| Isopropanol | 63.9 | | 63.9 | | 63.9 |
| Acetone | 21.9 | | 21.9 | | 21.9 |
| Purified water | 4.0 | | 4.0 | | 4.0 |

**[Table 10]**

| Test results of coated tablets | | | |
|---|---|---|---|
| S/L ratio of methacrylic acid copolymers | Acid resistance test (pH: 1.2, 2 hr) | Drop test | |
| | | (30 cm × 10) | (60 cm × 10) |
| 8:2 | 3/3 | 10/10 | 10/10 |
| 7:3 | 3/3 | 10/10 | 10/10 |
| 6:4 | 3/3 | 10/10 | 9/10 |

| | | | |
|---|---|---|---|
| Number of passed products/Number of tested products | | | |

### (Example 4)

A coating solution having a composition of Table 11 was sprayed onto core tablets manufactured in Referential Example 1, to thereby form, on each tablet, an inner coating layer so that the amount of polyvinyl alcohol-polyethylene glycol graft copolymer was adjusted to 8 mg/cm² with respect to the surface area of the core tablet.

**[Table 11]**

| Ingredients | (mass%) |
|---|---|
| Polyvinyl alcohol-polyethylene glycol graft copolymer | 20.0 |
| Purified water | 80.0 |

Subsequently, to the thus-manufactured coated tablets having a polyvinyl alcohol-polyethylene glycol graft copolymer inner coating layer thereon, a coating solution having a composition of Table 1 was sprayed, to thereby form, on each tablet, an outer coating layer so that the amount of methacrylic acid copolymer S was adjusted to 3 to 6 mg/cm² with respect to the surface area of the core tablet. The mean value of the resultant tablets hardness measured by a hardness tester PTB502 (product of PHARMA TEST) was about 270 N to about 300 N.

As is clear from Table 12 showing the test results, the thus-manufactured coated tablets were found to have a sufficient level of acid resistance required for enteric drug products and to exhibit good impact resistance.

**[Table 12]**

| Test results of coated tablets | | | |
|---|---|---|---|
| Amount of methacrylic acid copolymer S of outer layer (mg/cm²) | Acid resistance test (pH: 1.2, 2 hr) | Drop test | |
| | | (30 cm × 10) | (60 cm × 10) |
| 3 | 3/3 | 10/10 | 10/10 |
| 4 | 3/3 | 10/10 | 10/10 |
| 6 | 3/3 | 10/10 | 10/10 |

| | | | |
|---|---|---|---|
| Number of passed products/Number of tested products | | | |

### (Referential Example 4)

Polaprezinc (active ingredient) (60%), crystalline cellulose (24%), mannitol (10%), crospovidone (3%), and hydroxypropylcellulose (about 2%) were mixed and wet-granulated. The granulated product was dried and then mixed with magnesium stearate (about 1%). The mixture was compressed by a rotary tablet press, to thereby manufacture tablets each weighing about 1167 mg and containing 700 mg of active ingredient. The longer diameter and shorter diameter of each tablet are 20.5 mm and 9.5 mm, respectively. The mean value of the core tablet hardness measured by a hardness tester PTB502 (product of PHARMA TEST) was about 220 N.

### (Example 5)

A coating solution having a composition of Table 3 was sprayed onto core tablets manufactured in Referential Example 4, to thereby form, on each tablet, an inner coating layer so that the amount of hypromellose was adjusted to 8 mg/cm² with respect to the surface area of the core tablet.

Subsequently, to the thus-manufactured tablets having a hypromellose inner coating layer thereon, a coating solution having a composition of Table 1 was sprayed, to thereby form, on each tablet, an outer coating layer so that the amount of methacrylic acid copolymer S was adjusted to 4 mg/cm² or 6 mg/cm² with respect to the surface area of the core tablet. The mean values of the hardness of these two types of tablets measured by a hardness tester PTB502 (product of PHARMA TEST) were about 380 N and about 430 N, respectively.

As is clear from Table 13 showing the test results, the thus-manufactured coated tablets were found to have a sufficient level of acid resistance required for enteric drug products and to exhibit good impact resistance.

**[Table 13]**

| Test results of coated tablets | | | |
|---|---|---|---|
| Amount of methacrylic acid copolymer S of outer layer (mg/cm²) | Acid resistance test (pH: 1.2, 2 hr) | Drop test | |
| | | (30 cm × 10) | (60 cm × 10) |
| 4 | 3/3 | 10/10 | 10/10 |
| 6 | 3/3 | 10/10 | 10/10 |

| | | | |
|---|---|---|---|
| Number of passed products/Number of tested products | | | |

## Claims

1. An enteric coated tablet, comprising (A) a core tablet containing a medicinal ingredient and having a weight of 1,000 mg or more; (B) a coating layer containing a water-soluble polymer applied onto the surface of the core tablet, the water-soluble polymer coating layer (B) having no enteric property; and (C) an enteric coating layer dissolving at a pH of 7 or higher which is applied onto the surface of the coating layer containing a water-soluble polymer, wherein a sum of a polymer amount of the coating layer (B) and a polymer amount of the coating layer (C) is 10 to 18 mg/cm², a polymer amount of the coating layer (B) is 6 to 12 mg/cm², and a polymer amount of the coating layer (C) is 3 to 6 mg/cm².

2. The enteric coated tablet according to claim 1, wherein the core tablet (A) has a weight of 1,000 to 1,500 mg.

3. The enteric coated tablet according to claim 1 or 2, which has a medicinal ingredient content of 700 to 1,300 mg.

4. The enteric coated tablet according to any one of claims 1 to 3, wherein the water-soluble polymer is one or more members selected from the group consisting of water-soluble cellulose ethers.

5. The enteric coated tablet according to any one of claims 1 to 4, wherein the enteric coating layer dissolving at pH 7 or higher is a coating formed of one or more polymers selected from the group consisting of methacrylic acid copolymers.

6. The enteric coated tablet according to any one of claims 1 to 5, which has a total weight of 1,200 to 1,600 mg.

7. The enteric coated tablet according to any one of claims 1 to 6, wherein the medicinal ingredient is a therapeutic agent for a colonic disease.

8. The enteric coated tablet according to any one of claims 1 to 6, wherein the medicinal ingredient is a therapeutic agent for an inflammatory bowel disease.

9. The enteric coated tablet according to any one of claims 1 to 6, wherein the medicinal ingredient is 5-aminosalicylic acid.

10. The enteric coated tablet according to any one of claims 1 to 9, which has a ratio by polymer mass (B/C) of the coating layer (B) to the coating layer (C) of 1.0 to 2.5.

## Patentansprüche

1. Magensaftresistente Tablette, umfassend (A) eine Kerntablette, die einen medizinischen Inhaltsstoff enthält und ein Gewicht von 1.000 mg oder mehr aufweist; (B) eine Überzugsschicht, die ein wasserlösliches Polymer enthält, das auf die Oberfläche der Kerntablette aufgebracht ist, wobei die Überzugsschicht mit dem wasserlöslichen Polymer (B) keine magensaftresistente Eigenschaft aufweist; und (C) eine magensaftresistente Überzugsschicht, die sich bei einem pH von 7 oder höher löst, die auf die Oberfläche der das wasserlösliche Polymer enthaltenden Überzugsschicht aufgebracht ist, wobei die Summe der Menge des Polymers der Überzugsschicht (B) und der Menge des Polymers der Überzugsschicht (C) 10 bis 18 mg/cm² beträgt, die Menge des Polymers der Überzugsschicht (B) 6 bis 12 mg/cm² beträgt und die Menge des Polymers der Überzugsschicht (C) 3 bis 6 mg/cm² beträgt.

2. Magensaftresistente Tablette nach Anspruch 1, wobei die Kerntablette (A) ein Gewicht von 1.000 bis 1.500 mg aufweist.

3. Magensaftresistente Tablette nach Anspruch 1 oder 2, die einen Gehalt an medizinischem Inhaltsstoff von 700 bis 1.300 mg aufweist.

4. Magensaftresistente Tablette nach einem der Ansprüche 1 bis 3, wobei das wasserlösliche Polymer ein Element ist oder mehrere, ausgewählt aus der Gruppe, bestehend aus wasserlöslichen Celluloseethern.

5. Magensaftresistente Tablette nach einem der Ansprüche 1 bis 4, wobei die magensaftresistente Überzugsschicht, die sich bei pH 7 oder höher löst, eine Beschichtung ist, die aus einem oder mehreren Polymeren gebildet wird, ausgewählt aus der Gruppe, bestehend aus Methacrylsäure-Copolymeren.

6. Magensaftresistente Tablette nach einem der Ansprüche 1 bis 5, die ein Gesamtgewicht von 1.200 bis 1.600 mg aufweist.

7. Magensaftresistente Tablette nach einem der Ansprüche 1 bis 6, wobei der medizinische Inhaltsstoff ein therapeutisches Mittel für eine Darmerkrankung ist.

8. Magensaftresistente Tablette nach einem der Ansprüche 1 bis 6, wobei der medizinische Bestandteil ein therapeutisches Mittel für eine entzündliche Darmerkrankung ist.

9. Magensaftresistente Tablette nach einem der Ansprüche 1 bis 6, wobei der medizinische Inhaltsstoff 5-Aminosalicylsäure ist.

10. Magensaftresistente Tablette nach einem der Ansprüche 1 bis 9, die ein Verhältnis der Polymermasse (B/C) der Überzugsschicht (B) zu der Überzugsschicht (C) von 1,0 bis 2,5 aufweist.

## Revendications

1. Comprimé à enrobage entérique comprenant (A) un comprimé de cœur contenant un ingrédient médicinal et ayant un poids de 1000 mg ou plus ; (B) une couche d'enrobage contenant un polymère soluble dans l'eau appliqué sur la surface du comprimé de cœur, la couche d'enrobage en polymère soluble dans l'eau (B) n'ayant pas de propriété entérique ; et (C) une couche d'enrobage entérique se dissolvant à un pH de 7 ou plus qui est appliquée sur la surface de la couche d'enrobage contenant un polymère soluble dans l'eau, dans lequel la somme de la quantité de polymère de la couche d'enrobage (B) et de la quantité de polymère de la couche d'enrobage (C) est de 10 à 18 mg/cm², la quantité de polymère de la couche d'enrobage (B) est de 6 à 12 mg/cm², et la quantité de polymère de la couche d'enrobage (C) est de 3 à 6 mg/cm².

2. Comprimé à enrobage entérique selon la revendication 1, dans lequel le comprimé de cœur (A) a un poids de 1000 à 1500 mg.

3. Comprimé à enrobage entérique selon la revendication 1 ou 2, qui a une teneur en ingrédient médicinal de 700 à 1300 mg.

4. Comprimé à enrobage entérique selon l'une quelconque des revendications 1 à 3, dans lequel le polymère soluble dans l'eau est un ou plusieurs membres choisis dans le groupe constitué par les éthers de cellulose solubles dans l'eau.

5. Comprimé à enrobage entérique selon l'une quelconque des revendications 1 à 4, dans lequel la couche d'enrobage entérique se dissolvant à pH 7 ou plus est un enrobage formé d'un ou plusieurs polymères choisis dans le groupe constitué par les copolymères d'acide méthacrylique.

6. Comprimé à enrobage entérique selon l'une quelconque des revendications 1 à 5, qui a un poids total de 1200 à 1600 mg.

7. Comprimé à enrobage entérique selon l'une quelconque des revendications 1 à 6, dans lequel l'ingrédient médicinal est un agent thérapeutique pour une maladie du côlon.

8. Comprimé à enrobage entérique selon l'une quelconque des revendications 1 à 6, dans lequel l'ingrédient médicinal est un agent thérapeutique pour une maladie intestinale inflammatoire.

9. Comprimé à enrobage entérique selon l'une quelconque des revendications 1 à 6, dans lequel l'ingrédient médicinal est l'acide 5-aminosalicylique.

10. Comprimé à enrobage entérique selon l'une quelconque des revendications 1 à 9, qui a un rapport en masse de polymère (B/C) de la couche d'enrobage (B) sur la couche d'enrobage (C) de 1,0 à 2,5.
